# EUROPEAN PATENT SPECIFICATION

(11) **EP 1 007 095 B1**
(45) Date of publication and mention of the grant of the patent: **15.09.2004**
(21) Application number: 97925249.1
(22) Date of filing: 04.06.1997
(51) Int. Cl.: A61K 47/48, A61P 35/00

(54) **CHIMERIC TOXINS COMPRISING GnRH FOR TARGETED THERAPY**
CHIMÄRE TOXINE ENTHALTEND GnRH ZUR ZIELGERICHTETEN THERAPIE
TOXINES CHIMERES COMPRENANT DE LA GnRH POUR THERAPIE CIBLEE

(30) Priority: 04.06.1996 IL 11857096
(43) Date of publication of application: 14.06.2000
(73) Proprietor: YISSUM RESEARCH DEVELOPMENT COMPANY OF THE HEBREW UNIVERSITY OF JERUSALEM, Jerusalem 91042 (IL)
(72) Inventor: YARKONI, Shai, 44395 Kfar Saba (IL); NECHUSHTAN, Amotz, 47223 Ramat Hasharon (IL); LORBERBOUM-GALSKI, Haya, 97875 Jerusalem (IL); MARIANOVSKI, Irina, 91120 Jerusalem (IL)
(74) Representative: Benedum, Ulrich Max, Dr.
(86) International application number: PCT/IL1997/000180
(87) International publication number: WO 1997/046259

(56) References cited:
- WO-A-90/09799
- WO-A-93/15751
- WO-A-96/24675
- WO-A-97/22364
- NECHUSHTAN, AMOTZ ET AL: "Adenocarcinoma cells are targeted by the new GnRH -PE66 chimeric toxin through specific gonadotropin-releasing hormone binding sites" J. BIOL. CHEM. (1997), 272(17), 11597-11603 CODEN: JBCHA3;ISSN: 0021-9258, 1997, XP002051492
- RUSIECKI, V. K. ET AL: "GnRH -toxin chimera as chemosterilants: Synthesis and conjugation of GnRH analogs to truncated bacterial toxins" PEPT. 1994, PROC. EUR. PEPT. SYMP., 23RD (1995), MEETING DATE 1994, 765-766. EDITOR(S): MAIA, HERNANI L. S. PUBLISHER: ESCOM, LEIDEN, NETH. CODEN: 63MBAO, 1995, XP002051493

## Description

### Field of the invention

The present invention relates generally to therapeutic agents useful particularly in targeted cancer therapy but also in treating malignant carcinomas such as breast, colon, hepatic, ovarian and renal carcinomas and in treating benign tumors of the uterus, hyperplasia, endometriosis, BPH, polycystic disease of the breast and pituitary adenomas.

### Background of the invention

Gonadotropin releasing hormone (hereinafter called GnRH) participates in the hypothalamic - pituitary gonadal control of human reproduction. The involvement of GnRH has been demonstrated in several carcinomas. GnRH analogue treatment has been applied in breast, prostatic, pancreatic, endometrial and ovarian cancers (Kadar T. et al. Prostate 12: 229 - 307, 1988). These analogues suppress tumor cell growth in vitro and in vivo. The existence of GnRH binding sites has been revealed in the corresponding malignant cells and in well established cell lines (Emons G. et al. J.Clin.Endocrinol.Metab. 77: 1458 - 1464, 1993), though preliminary results suggest that the GnRE receptor involved may differ from the previously documented receptor (Kadar et al. Biochem. Biophs. Res. Comm. 189: 289 - 295, 1992).

Although GnRH binding sites have been demonstrated in a number of solid tumors and various carcinoma cell lines mainly derived from hormone dependant tissues, their existence in colon or renal carcinoma has not been previously documented. The presence of specific GnRH binding sites in colon, breast, prostate, ovarian endometrium, renal and liver carcinomas, is shown here. surprisingly, the specific GnRH binding sites are not limited to hormone-dependant tissues, as indicated by the marked killing of colon carcinoma, renal cell carcinoma and hepatocarcinoma cells.

WO 93/15751 describes various conjugates of gonadotropin releasing hormon (GnRH) and Pseudomonas exotoxin (PE) wherein the proteins moieties are chemically coupled via a linker group. These conjugates have also been used in the chemical sterilization of animals. The chemical conjugate of GnRH and PE can bind to the GnRH receptor present on gonadotrophs such as the luteinising hormone (LH) releasing cells of the pituitary gland. The binding then results in a killing of the cell which bears the gonadotropin receptor so that the pituitary gland will no longer produce hormones such as the luteinising hormone (LH) and the follicle stimulating hormone (FSH). This provides a chemical castrating effect and a number of sex-steroid dependent tumors respond to such hormonal manipulations.

### Object of the invention

It was therefore the object of the present invention to provide a new targeted toxin for a killing of another group of cells and which can be used for preparing a useful medicament.

### Summary of the invention

This object has been achieved by a targeted chimeric toxin molecule as claimed in claim 1 which can be obtained by fusion and expression of cDNA sequences encoding at least one cell-targeting moiety comprising a gonadotropin-releasing hormone (GnRH) and adapted to recognise cells bearing GnRH binding sites, and at least one cell-killing moiety essentially consisting of a mutated form of Pseudomonas exotoxin (PE) for a killing of cells bearing GnRH binding sites.

Preferred and other aspects of the invention are described in dependent claims 2 to 20 and comprise a method for the production of the targeted chimeric toxin, the use of the targeted chimeric toxin molecules for preparing a medicament and various pharmaceutical compositions.

More specifically the invention relates to Pseudomonas Exotoxin based chimeric toxins which bind to and attack neoplastic cells bearing gonadotropin releasing hormone binding sites. The present invention further relates to pharmaceutical compositions comprising as an active ingredient the above mentioned neoplastic cell targeted chimeric toxins. Furthermore the present invention relates to a method for the production of said chimeric toxins. These chimeric proteins are comprised of cell targeting moieties, which consist of gonadotropin releasing hormone homologues linked to cell killing moieties, which preferably consist of the bacterial toxin Pseudomonas Exotoxin A , and therefore they can recognize and destroy neoplastic cells bearing gonadotropin releasing hormone binding sites.

Targeting is a term for the selective delivery of chemotherapeutic agents to specific cell populations. It is possible to create chimeric molecules that possess cell targeting and cellular toxin domains. These chimeric molecules function as cell selective poisons by virtue of their capability to target selective cells and then kill those cells via their toxin component. Pseudomonas Exotoxin A (hereinafter called PE), a bacterial toxin used in construction of such chimeric proteins, acts by irreversibly arresting protein synthesis in eukaryotic cells, resulting in cell death.

The term "gonadotropin releasing hormone homologues" as used herein below relates to the gonadotropin releasing hormone gene itself or its analogues and antagonists.
Also described are salts of the described chimeric proteins of the present invention. The term "salts" includes both salts of carboxy groups as well as acid addition salts of amino groups of the protein molecule. The invention further relates to pharmaceutical compositions comprising the chimeric proteins as defined above together with a pharmaceutically acceptable inert carrier. The proteins of the present invention may be administered by methods known in the art for the administeration of proteins.

The present invention describes the construction, by the techniques of genetic enginering, of PE based chimeric toxins, aimed at targeting those neoplastic cells bearing GnRH binding sites. The chimeric toxins of the present invention are fusion proteins and, as such do not contain a chemical linker group (as disclosed in WO 93/15751). Therefore, they are completely different from the molecules described in the WO 93/15751.

Using different kinds of targeting moieties, a large number of immunotoxins have been generated in the last 20 years by chemical linking techniques or recombinant DNA technology. The size of these targeting moieties varies widely, ranging from large antibodies to small growth factors, cytokines and antibody fragments.

The ability of large chimeric proteins as the GnRH-PE constructions described in the present invention to target cells via a very small portion of the polypeptide (a peptide of ten amino acids is used as the targeting moiety of the present invention), and yet retain their original functions, namely binding and internalization, open up new possibilities in designing targeted immunotoxins.

Colon, breast, and prostate cancer - three out of the four major malignancies occuring in humans, together with ovarian, endometrium, renal and liver carcinomas, account for more than 50% of cancer related death. The presence of specific GnRH binding sites in all these cancers is suggests a more general role of GnRH and/or GnRH-like peptides in the malignant process.

If taken together, these results disclose what -could be considered the Achilles' heel of these malignant growths, a finding which could open up new routes in the fight against cancer.

In view of the efficient growth inhibition of the above mentioned cancer cells and their specificity vis-a-vis the non-target cells, the novel GnRH-PE chimeric toxins are promising candidates for cancer treatment.

The present invention relates particularly to targeted chimeric toxin molecules comprised of cell targeting moieties and cell killing moieties for recognizing and for destroying the neoplastic cells, wherein the cell targeting moieties consist of gcnadotropin releasing hormone homologues and the cell killing moieties consist of Pseudomonas Exotoxin A. The present invention further relates to pharmaceutical compositions containing as an active ingredient these targeted chimeric toxins and to a method for the production of these chimeric toxins. The present application also discloses a method for cancer therapy, treating malignant carcinoma cells and benign hyperplasia including uterine lyomyoma cells, extra uterian endometrial island cells, benign hyperplasia of prostate and breast and pituitary tunor adenoma cells, by the use of the above mentioned chimeric toxins.

### Detailed description of the invention

The present invention describes Pseudomonas Exotoxin A (PE) based chimeric toxins constructed by ligating an oligonucleotide encoding ten amino acids of a gonadotropin releasing hormone (GnRH) analog (GnRH coding sequence with tryptophane replacing glycine as the sixth amino acid) upstream to a mutated form of PE (domains I(mutated),II and III) thereby generating GnRH-PE66, and a ten amino acid synthetic GnRH oligomer(GnRH coding sequence with tryptophane replacing glycine as the sixth amino acid) ligated to domains II and III of the PE, thereby generating GnRH-PE40 protein.

The applications, potential markets and commercial advantages of the said chimeric proteins according to the present invention are listed:
These are two main applications:
   1) The malignant carcinomas:
      Breast, colon, hepatic, ovarian and renal carcinomas were all sensitive to GnRH - PE mediated cytotoxicity. Thus, the potential market for this new chimeric protein includes all carcinoma patients either as a first line of treatment or for patients in which other modalities of treatment had failed.
   2) The benign tumors of the uterus and hyperplasia:
      This group of pathalogies includes various tissues that are known to be sensitive to GnRH and thus can be targeted by the GnRH-PE chimeric proteins.

      a. Uterine - Uterine lyomyoma is the most common benign tumor in women. The uterine myomas are found to carry a large number of GnRH receptors. GnRH analogs are clinically used for down regulation and shrinkage of these myomas. The disadvantage of GnRH analogs is that these compounds can not be used for long periods and the myomas return to their original size after cessation of the treatment. The use of GnRH-PE for the destruction of the myomas can help to avoid what was considered to be iminant hysterectomies, as well as hemostatic drugs taken by these patients. The optional market includes women with fibroid uterus.
      b. Endometriosis - Endometrioma: The existence of endometrial tissue out of the uterus leads to the disease called endometriosis which can cause infertility, abdominal pain and even surgical emergencies.
         The endometrial islands are known to be very sensitive to hormonal changes. One of the therapeutic modalities found to be clinically effecient is the GnRH analog. Using GnRH-PE the endometrial activity of these islands can be arrested, thereby helping infertile couples as well as women who are undergoing laparotomy for the resection of these endometrial islands. The treatment of both the lyomyoma and the endometria can be administered systematically or locally by either ultra sonic or laparoscopic guided injection into the endometriomal peritoneal cavity or by a slow release into the uterine cavity.
      c. Benign Prostatic Hyperplasia (BPH):
         The prostatic cells are known to express GnRH receptors and prostatic cancer is successfully treated today with GnRH agonists. The BRH cause severe symptoms of dissuria urinary retention and sometimes can be treated only by prostatectomy.
         The use of GnRH-PE can therefor replace all prostatectomies procedures carried on prostate hyperplasia that is not malignant.
         The potential market is all the elderly men suffering from symptomatic prostate enlargement. GnRH-PE chimeric proteins may be administered systemically or transuterally.
      d. Polycystic disease of the breast:
         The mammary cells are also known to express the GnRH receptors. As in the case of BPH, the polycystic disease of the breast may be symptomatic, cause pain and may mimic breast carcinoma. The use of GnRH-PE may eliminate the need for numerous check-ups and needless mamographies and help woman suffering from breast pains and ***** of breast malignancy.
         Thhe potential market is a large population of woman in whom polycystic breast disease is diagnosed. GnRH-PE may be administered systemically.
      e. Pituitary adenoma: some of the pituitary adenomas are derived from gonadotropic cells. The pituitary adenoma, even though non-malignant, can cause a bad prognosis by causing local pressure on vital organs (eyes, brain stem). The trans-sphenoidal surgery used for the pituitary adenoma has many disadvantages including recurrency and neurological sequella. GnRH-PE may be aimed directly against the gonadotropic cells without damaging other functions of the pituitary gland. GnRH-PE chimeric toxin may be administered intra-thecally.

commercial advantages:
1. The wide variety of tumors that respond to the GnRH-PE chimeric protein.
2. The high selectivity that allows a large therapeutic range.
3. The use of GnRH as a targeting peptide makes the large population of postmenopausal women in whom the GnRH has no physiological role perfect candidates for the treatment.
4. Its high specificity allows systemic administration for obtaining a local effect.
5. The ability to eradicate small populations of cells in a tissue that will not be harmed by itself.

The proteins of the present invention may be administered by methods known in the art for the administeration of proteins.

Also included in the scope of the present invention are salts of the described chimeric proteins. The term "salts" includes both salts of carboxy groups as well as acid addition salts of amino groups of the protein molecule.

Salts of the carboxy group may be formed by methods known in the art and include both inorganic salts as well as salts with organic bases. The invention further relates to pharmaceutical compositions comprising the chimeric proteins as defined above together with a pharmaceutically acceptable inert carrier. The pharmaceutical composition may be administered by injection (intra-veneous, intra-articular, sub-cutaneous, intra-muscular, intra-peritoneal) toppical application, oral administration, sustained release, or by any other route including the enteral route.

The invention will now be described in detail by the following experiments and attached drawings and photographs. These experiments and figures do not intend to limit the scope of the invention but to demonstrate and clarify it only.

### Description of the Figures:

Figure 1: Construction and expression of the GnRH-PE66 chimeric toxin. A, SDS-PAGE gel and B, immunoblotting analysis of TGnRH-PE66 plasmid expression. Whole cell extract of the lysed bacteria (lane 1). Soluble fraction (lane 2). Insoluble fraction (lane 3). C, construction of TGnRH-PE66 plasmid.
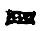 T7 promotor. 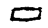 GnRH analogue peptide. 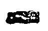 Ampicillin^{R}
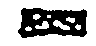 PE664glu. The numbers represent the corresponding amino acids.

Figure 2: The effect of increasing concentrations of GnRH-PE66 on various cell lines.
A: ■ SW-48 colon carcinoma, • HepG2 hepatocarcinoma, ▲ Caco2 colon carcinoma.
B: 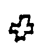 OVCAR3 ovarian carcinoma, 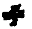 Hela cervix adenocarcinoma, ○ MDA breast carcinoma, × HT-29 colon carcinoma.
Figure 3: The effect of GnRH-PE66 on various primary cultures. A, colon carcinoma primary cultures established from three patients. B, renal cell carcinoma primary culture. C, breast carcinoma primary cultures established from four patients. D, ovarian carcinoma primary cultures established from two patients. E, metastases primary cultures established from the corresponding patients represented in A,C and D by the same symbols. ● colon carcinoma metastases. 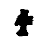 breast carcinoma metastases. 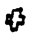 ,○ two ovarian carcinoma metastases. F, control cells: # leukocytes. ▲ bonemarrow. • fibroblasts. ∇ colon.
Figure 4: Histopathological diagnosis of primary cultures. A, anti-keratin positive staining of a colon primary culture. B, anti-desmin negative staining of a colon primary culture.
Figure 5: Displacement of [¹²⁵I] GnRH bound to membranes of SW-48 cells by: ○ GnRH-PE66. ■ GnRH analogue (des-Gly¹⁰,[d-Ala⁶]-LHRH).
Figure 6: Purification of GnRH-PE66.
   1 - protein marker. 2 - whole cell extract. 3 - soluble fraction. 4 - insoluble fraction after refolding. 5 - after DEAE - Sepharose column. 6 - after Sepharyl S-200 HR column.
Figure 7: Purification of GnRH-PE40:
   1 - protein marker. 2 - whole cell extract. 3 - soluble fraction. 4 - insoluble fraction after refolding. 5 - after DEAE - Sepharose column. 6 - after Sepharyl S-200 HR column.
Figure 8: Effects of GnRH-PE chimeric proteins on SW-48 colon carcinoma cell line:
   ■ GnRH-PE66 insoluble fraction after refolding.
   ▲ GnRH-PE66 purified protein.
   ● GnRH-PE40 purified protein.

### Experiments

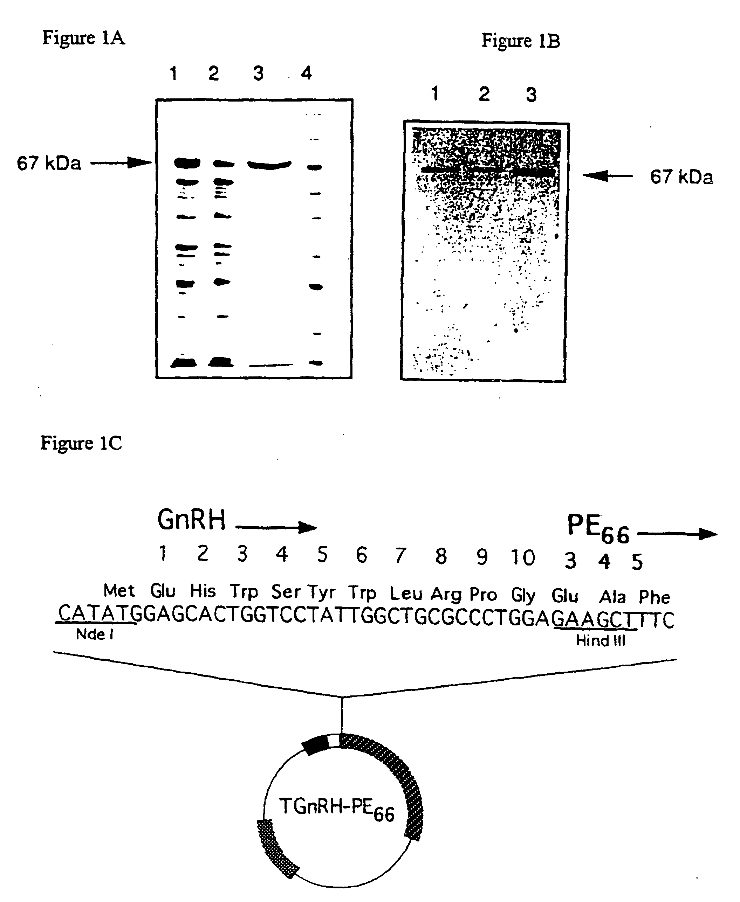

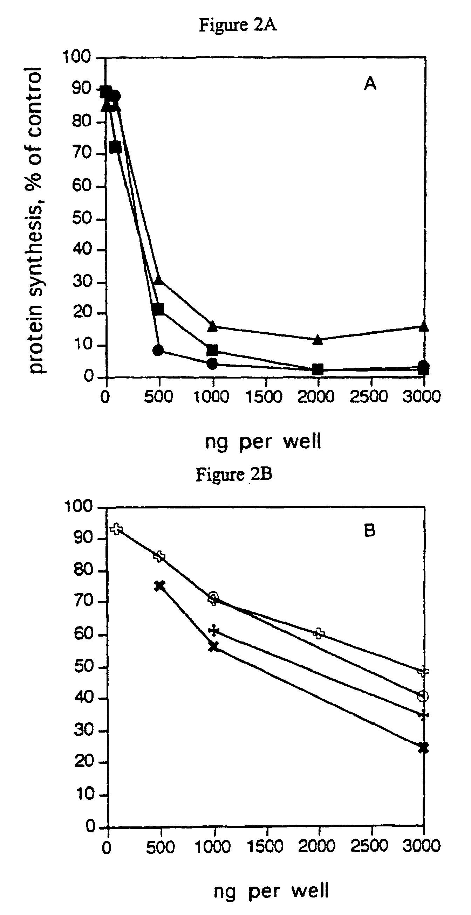

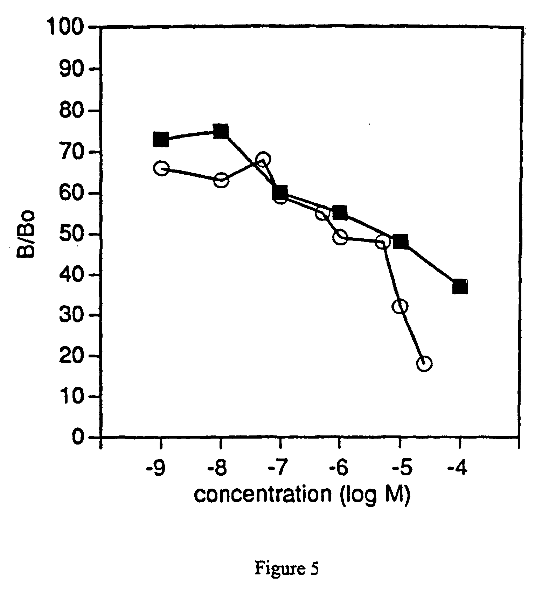

1. GnRH-PE66 chimeric toxin construction
A plasmid vector carrying the mutated full length PE gene (pJY3A1136-1,3) (Chandhary V., Jinno Y., Gall M., Fitzgerald D. and Patsan T. J. Biol. Chem. 256, 16306-16310, 1990) was cut with NdeI and Hind III. The insert, a 36 base pair synthetic oligomer consisting of the GnRH coding sequence with tryptophan replacing glycin as the sixth amino acid, was flanked by NdeI (5' end) and HindIII (3' end) restriction sites. The resulting TGnRH-PE66 plasmid was confirmed by restriction endonucleases digestion and DNA sequence analysis (Figure 1c).
2. TGnRH-PE40 piasmid construction
For construction of the GnRH-PE40 protein (GnRH-domains II and III of the PE), the TGnRH-PE66 plasmid vector (fig. 1c) was digested with NdeI and HamHI and ligated to a NdeI-BamHI 750bp fragment from the plasmid PHL-906 (Fishman A., Bar-Kana Y., Steinberger I. and Lorberboum-Galski H. Biochemistry 33, 6235-6243, 1994) along with an insert, i.e. the above-identified 36 base pair synthetic oligomer consisting of the GnRH coding sequence with tryptophan replacing glycin as the sixth amino acid, flanked by NdeI (5' end) and HindIII (3' end) restriction sites. The resulting TGnRH-PE40 plasmid was confirmed by restriction endonucleases digestion and DNA sequence analysis.
3. Protein expression
Protein expression method was the same for GnRH-PE40 and GnRH-PE66, unless mentioned otherwise. Escherichia coli strain BL21 (DE3) carrying the plasmid TGnRH-PE66 was grown in LB medium containing ampicillin (100µg/ml) and Escherichia coli strain BL21 ( DE3) carrying the plasmid GnRH-PE40 was grown in super LB medium containing ampicilin (50 µg/ml). After reaching an A600 value of 1.5 - 1.7, the cultures were induced 90 minutes for GnRH-PE66 and over night for GnRH-PE40, at 37°C with 1 mM isopropyl-d-thiogalactoside (IPTG). Cells were collected by centrifugation and the pellet was incubated at -70°C for several hours.
The frozen pellet was thawed and suspended in lysis buffer (50mM Tris HCl, pH 8.0, 1mM EDTA and lysozyme 0.2 mg/ml), followed by sonication (3 x 30 seconds) and centrifugation at 35,000 x g for 30 minutes. The supernatant (soluble fraction) was removed and the pellet (insoluble fraction) served as the source for the chimeric proteins and for their purification.
Analysis of the insoluble fraction by SDS/PAGE gel electrophoresis revealed a major band (70%) with an expected molecular mass of 67kDa, corresponding to the chimeric protein, and two major unrelated bacterial proteins of 42 and 28 kDa (fig. 1a). Immunoblotting with polyclonal antibodies against PE, confirmed these data (fig. 1.b).
4. Effect of the GnRH-PE66 chimeric proteins on various cell lines
In the experiments described below, the insoluble fraction of E.coli expressing cells was used as the source of the GnRH-PE66 chimeric protein.
The cytotoxic activity of GnRH-PE66 was tested in various established cell lines: SW-48 colon carcinoma, HepG2 hepatocarcinoma, Caco2 colon carcinoma, OVCAR3 ovarian carcinoma, Hela cervix adenocarcinoma, MDA breast carcinoma, HT- 29 colon carcinoma. Unless specified otherwise, all cell lines were maintained in RPMI 1640 medium, cultured in 100mm petri dishes in a humidified atmosphere of 5%CO₂/ 95% air at 37°C. HepG2 and Caco2 were maintained in Eagle's Minimal Essential Medium, Hela cells were maintained in Dulbecco's Modified Eagle's Medium. All media were supplemented with 10% fetal calf serum, 2mM L-glutamin, 100 units/ml of penicillin and 100µg/ml streptomycin. On day 0, cells (10⁴ in 0.2 ml culture medium) were seeded in 96 well tissue culture microplates and 24 hours later various concentrations of the GnRH-PE66 were added. After 24 hours incubation [³H]leucine [5µCi per well] was added for an additional 24 hours. At day 3, the plates were stored at -70°C for several hours, followed by a quick thawing at 37°C. Cells were harvested on filters and the incorporated radioactivity was measured with a beta counter. The chimeric protein was found to kill cells in a dose-dependent manner, with considerable variation between cell lines (table 1) ranging from the strong response of HepG2 hepatocarcinoma, SW-48 and Caca2 colon carcinomas (figure 2a) to the intermediate one of ovarian carcinoma OVCAR3, breast carcinoma MDA MB-231, colon carcinoma HT-29 and cervix adenocarcinoma Hela (figure 2b). Although cytotoxicity-was measured by inhibition of amino acid incorporation, cell death was reflected in cell number and\or cell necrosis 24 hours following the addition of the chimeric protein.
To confirm the specificity of GnRH-PE66 activity, two other PE based recombinant proteins, expressed and extracted under the same conditions, were used as controls. No substantial growth inhibition was exerted by either PE664Glu, encoded by the mutated full length PE gene, or by PIS2, an unrelated 80 bp sequence fused to PE664Glu. When 15 µg/ml of PE664Glu or PIS2 were added, protein synthesis ranged from a slight increase to 20% inhibition in the different cultures. Growth inhibition resulting from treatment with one of the two proteins was considered nonspecific. The results are given as percentages of the controls in which the cells were not exposed to any added protein (results are summarized in table 1 and in fig. 5).

**Table 1:**

| Cytotoxic activity of GnRH-PE66 on various cell lines. | | |
|---|---|---|
| Cell line | Origin | ID₅₀ (µg total protein/well)* |
| Caco2 | Colon carcinoma | 0.4 |
| HT-29 | Colon carcinoma | 1.2 |
| SW-48 | Colon carcinoma | 0.3 |
| OVCAR3 | Ovarian carcinoma | 3 |
| MDA MB-231 | Breast carcinoma | 2.3 |
| Hela | Cervix adenocarcinoma | 1.8 |
| HepG2 | Hepatocarcinoma | 0.3 |

| | | |
|---|---|---|
| * The ID₅₀ values show the effect of the insoluble fraction enriched with the chimeric protein. | | |

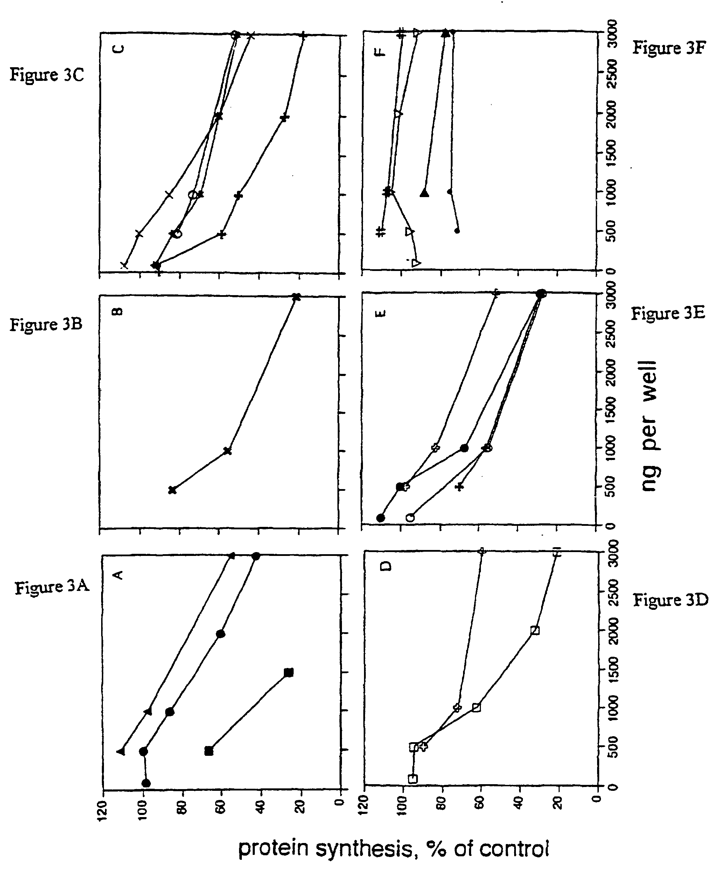

5. The effect of GnRH-PE66 on various primary cultures'
In order to evaluate the cytotoxic effect of the chimeric proteins of the invention on cells similar to the original in vivo tumors as closely as possible and to exclude the possibility that the GnRH-PE66 cytoxicity was a characteristic developed by cells upon prolonged passages, primary cultures were established.
Fresh tissue specimens were obtained from various cancer patients undergoing therapeutic debulking procedures. Control specimens were obtained from donors or patients undergoing diagnostic or therapeutic procedures for non - malignant diseases. All tissue specimens were washed several times with Leibovitz (L15) medium, and extensively cut with a scalpel. The preparations were then enzymatically proteolysed for 2 hours at 37°C with gentle shaking in Leibovitz medium containing collagenase type I (200u/ml), hyaluronidase (100u/ml), penicillin (1000units/ml), streptomycin (1mg/ml), amphotericin B (2.5 µg/ml) and gentamycin (80 µg/ml). Tissue preparations were centrifuged 10 minutes at 200 x g and the pellets were suspended in RPMI 1640 medium, supplemented with 10% fetal calf serum, penicillin (100u/ml) and streptomycin (100µg/ml) and plated in 100mm petri dishes. Cells were grown for one to three weeks to a density of 8 x 10⁶ cells and histopathological diagnoses and cytotoxic assays were performed. Normal leukocytes from peripheral blood and bone marrow aspirates for cytotoxic assays were obtained by diluting whole blood in one volume of phosphate - buffered saline. The diluted sample was placed over an equal volume of Ficoll - Paque and centrefuged for 10 minutes at 200 x g. The cells were resuspended and plated in RPMI 1640 medium containing 20% fetal calf serum, 4 mM 1 - glutamine, 50 µM β mercaptoethanol, non essential amino acids, 1mM sodium pyruvate, penicillin (100 units/ml) and streptomycin (100µg/ml).
The cytotoxic effect of the chimeric protein was variable (table 2) with up to three-fold differences in ID50 observed in colon, breast and ovarian primary cultures originated from different patients (figures 3a,c and d respectively).

**Table 2:**

| Cytotoxic effect of GnRH-PE66 on various primary cultures | |
|---|---|
| Origin | ID₅₀ (µg total protein/well)^{a} |
| Colon carcinoma | 0.8 - 2.5^{b} |
| Renal cell carcinoma | 1.2 |
| Breast carcinoma | 1 - 3^{c} |
| Ovarian carcinoma | 1.6 - 3^{d} |
| Bladder carcinoma | no effect |
| | |
| Control cells: | |
| Colon | no effect^{e} |
| Fibroblasts | " " |
| Bone marrow | " " |
| Leukocytes | " " |

| | |
|---|---|
| ^{a} The ID₅₀ values show the effect of the insoluble fraction enriched with the chimeric protein | |
| ^{b} n=3 | |
| ^{c} n=4 | |
| ^{d} n=2 | |
| ^{e} increasing concentration of GnRH-PE66 did not affect cell grow | |

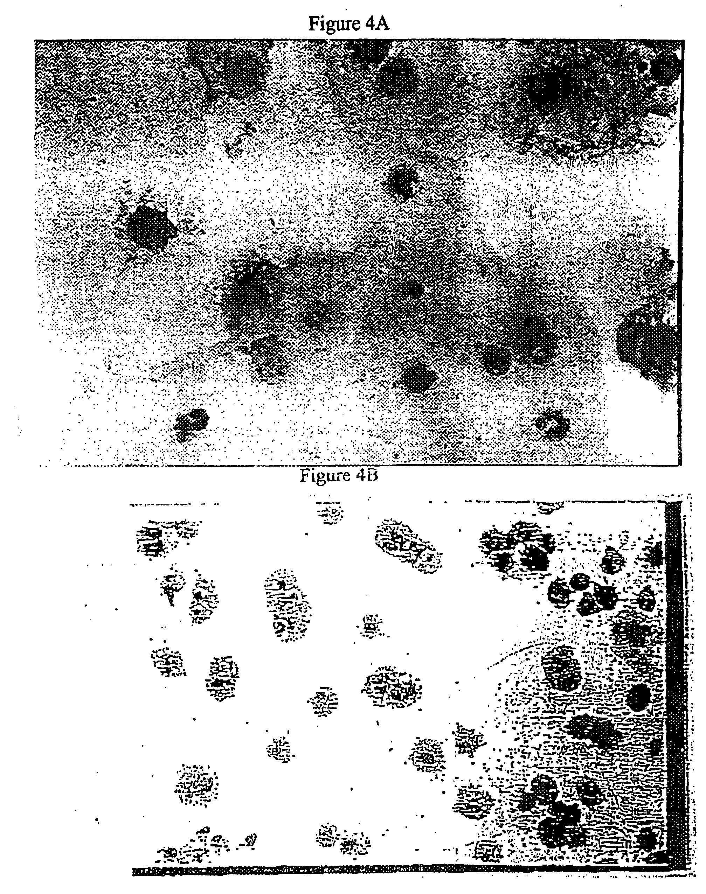

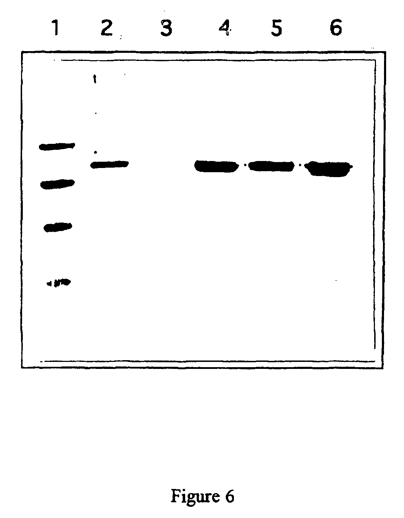

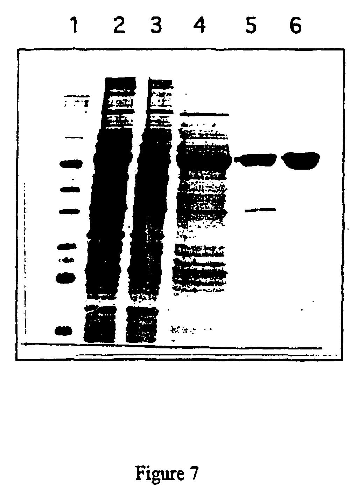

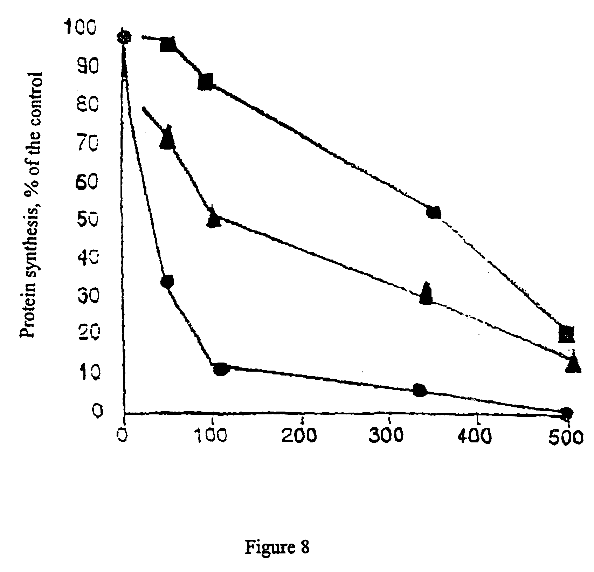

In cases where metastasis biopsies could also be obtained, cultures of primary tumors alongside with the metastasis were examined for GnRH-PE66 cytotoxicity. The metastatic cells responded in the same manner, and their ID₅₀ were even lower than those of the primary tumors. This may be explained by the high homogeneity of the metastasis culture compared with that of the primary culture.
GnRH-PE66 was also tested on cultures of benign colon peripheral blood bone marrow and skin fibroblasts from healthy donors. The addition of up to 15µg/ml of the chimeric protein did not result in any measurable dose dependant killing (fig 3f).
6. Histopathological diagnosis of primary cultures
One of the basic questions regarding the veracity of the primary cultures assays is of the epithelial origin of the cells. The tendency of cells in primary culture to lose their epithelial morphology has been described in several carcinomas. To confirm the absence of any substantial amount of "contaminating" fibroblasts, differential staining was performed.
Cells were stained as follows: 10,000 cells were plated on a microscope slide using a cytospin, followed by several minutes incubation at room temperature. Dried slides were fixed by soaking in -20°C cold methanol for 15 minutes and in -20°C cold acetone for a few seconds. Slides were kept at -20°C until staining. staining was carried out with anti-desmin and anti-keratin antibodies to distinguish fibroblast from epithelial cells, respectively. This staining indicated that the vast majority of the cells a 80-100%) were indeed epithelial, even in cases where the cultures exhibited a fibroblast-like shape (fig 4).
Further confirmation was achieved by staining with specific anti tumor marker antigens according to the type of cancer.
Formalin fixed sections from the original tumors and the primary cultures cells displayed the same pattern and intensity of staining.
7. Specific binding by GnRH-PE66
To support the findings that colon carcinoma cell lines and primary cultures can be targeted and killed by GnRH-PE66, the ability of plasma membrane fractions from a colon carcinoma cell line to specifically bind GnRH, was examined. The addition of increasing concentrations of GnRH-PE66 chimeric toxin resulted in dose - related displacement of the ¹²⁵I - GnRH bound to these membranes. A semiconfluent 100mm dish of the SW-48 colon carcinoma cell line was washed and the cells were scraped off the plate with a rubber policeman. The collected cells were homogenized in ice - cold assay buffer (10mM Tris - HCl, pH 7.6, 1mM dithiothreitol, 0.15% bovine serum albumin, 1mM EDTA) and centrifuged at 250 x g for 15 minutes (4°C). The resulting pellet was discarded and the supernatant was centrifuged at 20,000 x g for 30 minutes (4°C). The plasma membrane pellet was resuspended in cold assay buffer. Aliquots containing 70 µg plasma membrane protein in a final volume of 100µl, were incubated for 2 hours on ice with 6 x 10⁻⁶M (240,000 cpm) ¹²⁵I-GnRH either in the presence or absence of (10⁻⁴ - 10⁻¹⁰M) unlabeled GnRH authentic peptide and analog (des - Gly,[d-Ala)-LHRH) or (2.5 x 10⁻⁵ - 10⁻⁹M) GnRH-PE66 chimeric toxin. Following incubation, samples were washed through Whatman GF/C filters with 10 ml of cold assay buffer and counted in a gamma counter.
The addition of increasing concentrations of GnRH-PE66 chimeric toxin resulted in dose related displacement of the ¹²⁵I-GnRH bound to these membranes. Unlabeled authentic GnRH peptide and the analogue des-Gly10 [D-Ala6]-LHRH produced similar results. As can be seen in figure 5, binding of the labeled GnRH to SW-48 colon carcinoma cell line was specific and displacement by the GnRH-PE66 chimeric toxin was as efficient as that by the GnRH analogue peptide. There was 37% non specific binding.
8. GnRH-PE40 and GnRH-PE66 purification
The pellet of the insoluble fraction was suspended and stirred on ice in denaturation buffer (6M guanidium HCl, 0.1 M Tris HCl, pH 8.6 1mM EDTA 0.05M NaCl and 10mM DDT). After an additional centrifugation, the reduced and denatured protein was diluted 1:100 in refolding buffer (50mM Tris HCl, pH 8, 1mM EDTA, 0.25M NaCl, 0.25 M L-arginine and 5mM DTT) and kept at 4°C for 48 hours. Refolded protein solutions were diluted to 8 ms in TE20 buffer (20mM Tris pH 8.0, 1mM EDTA). DEAE Sepharose was added and stirred for half an hour at 4°C before being packed onto a column. Washing of the column was done with 80mM NaCl, in TE20 buffer for GnRH-PE66 and 50mM NaCl in TE20 buffer for GnRH-PE40. Elution was preformed with the linear gradient of 2 x 200ml of 0.08 - 0.35M NaCl, in TE20 (20mM Tris pH 8.0, 1mM EDTA) buffer. The peak fractions were pooled, 0.5M L-arginine was added and stirred cell was used for concentration. (fig. 2 and fig. 3). 3ml of the pooled fractions from the ion exchange column were loaded onto a Sephacryl S-200 HR gel filtration column, in 0.5M NaCl, 0.15M K -phosphate buffer, pH 6.0. The peak fractions were pooled, dialyzed against phosphate saline buffer and kept in aliquotes at -20°C. Purification of GnRH-PE66 and GnRH-PE40 is demonstrated in figures 6 and 7 respectively.
9. Effect of highly purified GnRH-PE chimeric proteins on SW-48 colon carcinoma cell line
The cytotoxic activity of the purified GnRH-PE66 and GnRH-PE40 on SW-48 colon carcinoma cell line was assessed by measuring the inhibition of protein synthesis. The chimeric proteins were found to kill cells in a dose dependent manner.
The ID₅₀ of the purified GnRH-PE66 chimeric toxin was two to three times lower than the refolded insoluble fraction.
The ID₅₀ of the GnRH-PE40 purified protein was three to four times lower than the purified GnRH-PE66 (fig. 8).

## Claims

1. Targeted chimeric toxin molecule obtained by fusion and expression of cDNA sequences encoding
at least one cell-targeting moiety comprising a gonadotropin-releasing hormone (GnRH) and adapted to recognize specifically cells bearing GnRH binding sites, and
at least one cell-killing moiety essentially consisting of a mutated form of Pseudomonas Exotoxin for killing specific cells bearing GnRH binding sites.

2. Targeted chimeric toxin molecule as claimed in claim 1, comprising a targeting moiety which recognizes GnRH binding sites of cells selected from the group consisting of malignant adenocarcinoma cells, benign uterine lyomyoma cells, endometrial island cells and pituitary tumor adenoma cells.

3. Targeted chimeric toxin molecule as claimed in claim 1 or 2, obtained by fusion of an oligonucleotide, encoding ten amino acids of a GnRH analog as shown in Figure 1c, to a mutated DNA fragment of the full length Pseudomonas Exotoxin to obtain GnRH-PE66.

4. Targeted chimeric toxin molecule as claimed in claim 1 or 2, obtained by fusion of an oligonucleotide encoding ten amino acids of a GnRH analog as shown in Figure 1c to a DNA fragment comprising domains II and III of the Pseudomonas Exotoxin, to obtain GnRH-PE40.

5. A plasmid comprising a DNA sequence encoding a targeted chimeric toxin as claimed in any preceding claim 1 to 4.

6. A method for the production of targeted chimeric toxin molecules as defined in claim 3, comprising the step of ligating an oligonucleotide encoding ten amino acids of a GnRH analog upstream to a DNA fragment encoding a mutated Pseudomonas exotoxin to obtain GnRH-PE66.

7. A method for the production of targeted chimeric toxin molecules as defined in claim 4, comprising the step of ligating an oligonucleotide encoding ten amino acids of a GnRH analog upstream to a DNA fragment encoding domains II and III of Pseudomonas Exotoxin to obtain GnRH-PE40.

8. Pharmaceutical composition comprising as active substance targeted chimeric toxin molecules as claimed in any preceding claim 1 to 4.

9. Use of targeted chimeric toxin molecules as claimed in any claim 1 to 4 for preparing a medicament for treatment of malignant carcinomas and benign tumors.

10. Use as claimed in any claim 1 to 4 for preparing a medicament for systemic administration or transcervical washing of the endometrial cavity.

11. Use of targeted chimeric toxin molecules as claimed in any claim 1 to 4 for preparing a medicament for treating endometriosis.

12. Use as claimed in claim 10 for preparing a medicament for peritoneal washing or ultrasonic guided or laparoscopic intra-endometrial injection or systemic administration.

13. Use of targeted chimeric toxin molecules as claimed in any claim 1 to 4 for preparing a medicament for treatment of uterine myomas.

14. Use as claimed in claim 13 for preparing a medicament for trans-cervical washing of the endometrial cavity.

15. Use of targeted chimeric toxin molecules as claimed in any claim 1 to 4 for preparing a medicament for treatment of pituitary adenomas.

16. Use as claimed in claim 15 for preparing a medicament for intra-thecal administration.

17. Use of targeted chimeric toxin molecules as claimed in any claim 1 to 4 for preparing a medicament for treatment of Benign Prostatic Hyperplasia (BPH).

18. Use as claimed in any claim 17 for preparing a medicament for systemic or trans-uteral administration.

19. Use of chimeric toxins as claimed in any claim 1 to 4 for preparing a medicament for treatment of polycystic breast disease.

20. Use as claimed in claim 19 for preparing a medicament for systemic administration.

## Patentansprüche

1. Zielgerichtete Toxinmolekülchimäre, erhalten durch Fusion und Expression von cDNA-Sequenzen, welche für
mindestens einen Zellentargeting-Rest kodieren, der Gonadotropin-Freisetzungshormon (GnRH) enthält und der spezifisch Zellen erkennt, die GnRH-Bindestellen tragen, und
mindestens einen Zellgiftrest, der im Wesentlichen aus einer mutierten Form von Pseudomonasexotoxin besteht und der spezifisch Zellen tötet, die GnRH-Bindestellen tragen.

2. Zielgerichtete Toxinmolekülchimäre nach Anspruch 1, umfassend einen Targetingrest, der GnRH-Bindestellen auf Zellen erkennt, welche ausgewählt sind aus der Gruppe maligne Adenokarzinomzellen, benigne Uterus-Lymphomzellen, Endometrium-Inselzellen und Tumoradenomzellen des Hypophysenvorderlappens.

3. Zielgerichtete Toxinmolekülchimäre nach Anspruch 1 oder 2, erhalten durch Fusion eines Oligonucleotids, das zehn Aminosäuren eines GnRH-Analogs gemäß Fig. 1c kodiert, mit einem mutierten DNA-Fragment von Pseudomonasexotoxin ganzer Länge, wobei man GnRH-PE66 erhält.

4. Zielgerichtete Toxinmolekülchimäre nach Anspruch 1 oder 2, erhalten durch Fusion eines Oligonucleotids, das zehn Aminosäuren eines GnRH-Analogs gemäß Fig. 1c kodiert, mit einem DNA-Fragment, welches die Domänen II und III aus Pseudomonasexotoxin umfasst, wobei man GnRH-PE40 erhält.

5. Plasmid, umfassend eine DNA-Sequenz, die ein zielgerichtete Toxinchimäre nach einem der Ansprüche 1 bis 4 kodiert.

6. Verfahren zur Herstellung von zielgerichteten Toxinmolekülchimären nach Anspruch 3, umfassend den Schritt des Legierens eines Oligonucleotids, das zehn Aminosäuren von einem GnRH-Analog kodiert, stromaufwärts von einem DNA-Fragment, das mutiertes Pseudomonasexotoxin kodiert, wobei man GnRH-PE66 erhält.

7. Verfahren zur Herstellung von zielgerichteten Toxinmolekülchimären nach Anspruch 4, umfassend den Schritt des Legierens eines Oligonucleotids, das zehn Aminosäuren von einem GnRH-Analog kodiert, stromaufwärts von einem DNA-Fragment, das die Domänen II und III von Pseudomonasexotoxin kodiert, wobei man GnRH-PE 40 erhält.

8. Pharmazeutische Zusammensetzung, die als Wirksubstanz zielgerichtete Toxinmolekülchimäre nach einem der Ansprüche 1 bis 4 enthält.

9. Verwendung von zielgerichteten Toxinmolekülchimären nach einem der Ansprüche 1 bis 4 zur Herstellung eines Medikaments zur Behandlung von malignen Karzinomen und benignen Tumoren.

10. Verwendung nach einem der Ansprüche 1 bis 4 zur Herstellung eines Medikaments für eine systemische Verabreichung oder zum transzervikalen Waschen des Endometrium-Hohlraums.

11. Verwendung von zielgerichteten Toxinmolekülchimären nach einem der Ansprüche 1 bis 4 zur Herstellung eines Medikaments zur Fachbehandlung von Endometriosis.

12. Verwendung nach Anspruch 10 zur Herstellung eines Medikaments für eine peritoneale Waschung oder eine Ultraschall-geführte oder laparoskopische intra-endometrische Injektion oder zur systemischen Verabreichung.

13. Verwendung von zielgerichteten Toxinmolekülchimären nach einem der Ansprüche 1 bis 4 zur Herstellung eines Medikaments zur Behandlung Uterus-Myomen.

14. Verwendung nach Anspruch 13 zur Herstellung eines Medikaments für eine transzervikale Waschung des Endometrium-Hohlraums.

15. Verwendung von zielgerichteten Toxinmolekülchimären nach einem der Ansprüche 1 bis 4 zur Herstellung eines Medikaments zur Behandlung von Adenomen des Hypophysenvorderlappens.

16. Verwendung nach Anspruch 15 zur Herstellung eines Medikaments zur intrathekalen Verabreichung.

17. Verwendung von zielgerichteten Toxinmolekülchimären nach einem der Ansprüche 1 bis 4 zur Herstellung eines Medikaments zur Behandlung von benigner Prostatahyperplasie (BPH).

18. Verwendung nach Anspruch 17 zur Herstellung eines Medikaments zur systemischen oder transuteralen Verabreichung.

19. Verwendung von Toxinchimären nach einem der Ansprüche 1 bis 4 zur Herstellung eines Medikaments zur Behandlung einer polyzystischen Brusterkrankung.

20. Verwendung nach Anspruch 19 zur Herstellung eines Medikaments zur systemischen Verabreichung.

## Revendications

1. Une molécule de toxine chimère cible obtenue par la fusion et l'expression de séquences d'ADN complémentaire codant au moins un fragment ciblant une cellule, comprenant gonadolibérine (GnRH) et adaptée pour reconnaître des cellules supportant de façon spécifique des sites de liaison GnRH, et au moins un fragment permettant de tuer des cellules, constituée essentiellement d'une forme mutée de l'exotoxine de Pseudomonas afin de tuer des cellules spécifiques supportant des sites de liaison GnRH.

2. Une molécule de toxine chimère cible comme revendiqué dans la revendication 1, comprenant un fragment ciblant qui reconnaît des sites de liaison GnRH de cellules choisies à partir du groupe constitué des cellules malignes d'adénocarcinome, des cellules utérines bénignes de lyomyoma, des cellules endométriales isolées et des cellules de tumeur d'adénome pituitaire.

3. Une molécule de toxine chimère cible comme revendiqué dans la revendication 1 ou 2, obtenue par la fusion d'une oligonucléotide, codant dix acides aminés d'un GnRH de façon similaire à celle montrée sur la figure 1c, en un fragment ADN muté d'exotoxine de Pseudomonas dans toute sa longueur pour obtenir un GnRH-PE66.

4. Une molécule de toxine chimère cible comme revendiqué dans la revendication 1 ou 2, obtenue par la fusion d'un oligonucléotide codant dix acides aminés d'un GnRH de façon similaire à celle montrée sur la figure 1C, en un fragment ADN comprenant les domaines II et III de l'exotoxine de Pseudomonas afin d'obtenir un GnRH-PE40.

5. Un plasmide comprenant une séquence ADN codant une toxine chimère cible, comme revendiqué dans n'importe laquelle des revendications 1 à 4.

6. Un procédé pour la fabrication de molécules de toxines chimères cibles comme défini dans la revendication 3, comprenant les étapes de lier un oligonucléotide codant dix acides aminés d'un GnRH de façon similaire en amont par rapport à un fragment ADN codant une exotoxine de Pseudomonas mutée afin d'obtenir un GnRH-PE66.

7. Un procédé pour la fabrication de molécules de toxines chimères ciblées comme défini dans la revendication 4, comprenant l'étape de lier un oligonucléotide codant dix acides aminés d'un GnRH de façon similaire en amont par rapport à un fragment ADN codant des domaines II et III d'exotoxine de Pseudomonas afin d'obtenir un GnRH-PE40.

8. Une composition pharmaceutique comprenant une substance active de molécules de toxines chimères cibles comme revendiqué dans n'importe laquelle des revendications précédentes 1 à 4.

9. L'utilisation de molécules de toxines chimères ciblées comme revendiqué dans n'importe laquelle des revendications 1 à 4 pour la préparation d'un médicament destiné au traitement d'un carcinome malin et des tumeurs bénignes.

10. L'utilisation comme revendiqué dans n'importe laquelle des revendications 1 à 4 pour la préparation d'un médicament pour l'administration systématique ou le lavage trans-cervical de la cavité endométriale.

11. L'utilisation de molécules de toxines chimères ciblées comme revendiqué dans n'importe laquelle des revendications 1 à 4 pour la préparation d'un médicament destiné au traitement de l'endométriose.

12. L'utilisation comme revendiqué dans la revendication 10 pour la préparation d'un médicament destiné au lavage péritonéal ou à un guidage par ultrasons ou une injection laparoscopique intra-endométrial ou à une administration systémique.

13. L'utilisation de molécules de toxines chimères ciblées comme revendiqué dans n'importe laquelle des revendications 1 à 4 pour la préparation d'un médicament destiné au traitement des myomes de l'utérus.

14. L'utilisation comme revendiqué dans la revendication 13 pour la préparation d'un médicament destiné au lavage trans-cervical de la cavité endométriale.

15. L'utilisation de molécules de toxines chimères ciblées comme revendiqué dans n'importe laquelle des revendications 1 à 4 pour la préparation d'un médicament destiné au traitement d'adénomes pituitaires .

16. L'utilisation comme revendiqué dans la revendication 15 pour la préparation d'un médicament destiné à une administration intrathécale.

17. L'utilisation de molécules de toxines chimères ciblées comme revendiqué dans n'importe laquelle des revendications 1 à 4 pour la préparation d'un médicament destiné au traitement d'une hyperplasie prostatique bénigne (BPH).

18. L'utilisation comme revendiqué dans la revendication 17 pour la préparation d'un médicament destiné à une administration systémique ou trans-utérine.

19. L'utilisation de toxines chimères comme revendiqué dans n'importe laquelle des revendications 1 à 4, pour la préparation d'un médicament destiné au traitement d'une maladie polycystique de la poitrine.

20. L'utilisation comme revendiqué dans la revendication 19 pour la préparation d'un médicament destiné à une administration systémique.
